(19) Europäisches Patentamt
European Patent Office
Office européen des brevets

(11) Publication number: 0 005 769
B1

(12) **EUROPEAN PATENT SPECIFICATION**

(45) Date of publication of patent specification: 21.04.82

(21) Application number: 79101517.5

(22) Date of filing: 18.05.79

(51) Int. Cl.[3]: C 07 C 27/14, C 07 C 57/04, C 07 C 47/22, B 01 J 27/18

(54) **A process for producing methacrolein and methacrylic acid.**

(30) Priority: 31.05.78 JP 64319/78
23.06.78 JP 75417/78

(43) Date of publication of application:
12.12.79 Bulletin 79/25

(45) Publication of the grant of the patent:
21.04.82 Bulletin 82/16

(84) Designated Contracting States:
BE DE FR GB IT NL

(56) References cited:
DD - A - 93 759
DD - A - 124 473
DE - A1 - 2 441 109
DE - A1 - 2 645 363
DE - A1 - 2 704 991
DE - A1 - 2 739 779
FR - A1 - 2 302 993
FR - A1 - 2 277 071
GB - A - 1 235 380
GB - A - 1 456 916
GB - A - 1 464 711

(73) Proprietor: NIPPON KAYAKU KABUSHIKI KAISHA
2-1, Marunouchi-1-chome Chiyoda-ku
Tokyo (JP)

(72) Inventor: Matsumoto, Mutsumi
No.239 Iwahana-cho
Takasaki-shi, Gunma-ken (JP)
Inventor: Wada, Kouichi
No.239 Iwahana-cho
Takasaki-shi, Gunma-ken (JP)
Inventor: Sudo, Atsushi
No.340-4 Haraichi
Annaka-shi, Gunma-ken (JP)

(74) Representative: UEXKÜLL & STOLBERG
Patentanwälte
Beselerstrasse 4
D-2000 Hamburg 52 (DE)

(56) References cited:
GB - A - 1 473 035
GB - A - 1 487 765
GB - A - 1 491 529
GB - A - 1 492 185
GB - A - 1 498 595
US - A - 3 925 464

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 005 769 B1

# A process for producing methacrolein and methacrylic acid

This invention relates to a process for producing methacrolein and methacrylic acid by oxidizing isobutylene and/or tertiary butanol with molecular oxygen or a molecular oxygen-containing gas in the vapor phase in the presence of the specific type catalyst.

Most of prior art processes proposed for producing unsaturated monocarboxylic acids from corresponding olefins consist of two steps: In the first step, corresponding aldehydes are produced by the use of a catalyst; and, in the second step, the unsaturated aldehydes are converted to corresponding unsaturated carboxylic acids by the use of a catalyst different from one used in the first step. Only a few catalysts are known which are effective for preparing unsaturated monocarboxylic acid by oxidizing corresponding unsaturated hydrocarbon by a single step, but when these catalysts are used, the yield of the unsaturated monocarboxylic acid is generally very low. Especially few catalysts are known for producing methacrylic acid by oxidation of isobutylene in one step, and these have a disadvantage that the yield of methacrylic acid is very low.

The inventors of the present application have discovered that the catalyst according to this invention shows high activity and high selectivity even at low temperature in the oxidation of isobutylene and/or tertiary butanol to methacrylic acid.

This invention relates to a process for producing methacrolein and methacrylic acid by the vapor phase oxidation of isobutylene and/or tertiary butanol with molecular oxygen or a molecular oxygen-containing gas in the presence of the catalyst represented by the following formula:

$$Mo_aV_b1P_c X_dO_e$$

wherein X represents at least one of the elements.

Cu, Ag, Zn, Cd, Al, Ge, Sn, Pb, Bi, Mg, Ca, Sr, Ba, Ti, Zr, Nb, Ta, W, Mn, Fe, Co, Ni, Ce, Th, Re, Cr, B, K, Rb, or Cs, and a, b, c, d and e represent the atomic ratio of the elements where, *a* is 10, *b* is a number of 6 or less than 6 excluding O, preferably 0.5 to 3, *c* is a number of 0.5 to 10, preferably 0.5 to 3, *d* is a number of 0.01 to 5, preferably 0.01 to 2.0, and *e* is a number determined depending on the valency and atomic ratio of the other elements present and is usually a number of 35 to 80).

From the point of view of the formula, the preferred catalyst is the one represented by the following formula:

$$Mo_{a'}\ V_{b'}\ P_{c'}\ Cu_{d'}\ X'_{e'}\ O_f$$

wherein X' represents at least one of the elements tin, thorium, aluminium, germanium, nickel, iron, cobalt, potassium, rubidium, cesium, zinc, titanium, lead, rhenium, zirconium, chromium and bismuth, and a', b', c', d', e' and f' represent the atomic ratio of the respective elements, where *a'* is 10, *b'* is a number of 6 or less than 6 excluding O, preferably 0.5 to 3, *c'* is a number of 0.5 to 10, preferably 0.5 to 3.0, *d'* is a number of 3 or less than 3 excluding O, preferably 0.01 to 1.0, *e'* is a number of 3 or less than 3 excluding O, preferably 0.01 to 1.0, provided that $d' + e'$ is a number of 0.01 to 5 and *f'* is a number determined depending on the valency and atomic ratio of the other elements present and is usually a number of 35 to 80).

The catalyst of the present invention can be prepared by the general methods for preparing the known oxidation catalysts such as oxide catalysts or catalysts having heteropoly-acid structure or heteropoly-acid salt structure.

There is no limitation to select starting materials to be used for the preparation of the catalyst and thus various materials are usable. The starting materials usable for the molybdenum component include, for example, ammonium molybdate, molybdic acid, molybdenum oxide and the like. The starting materials usable for the vanadium component include, for example, ammonium metavanadate, vanadium pentoxide and the like. The starting materials usable for the phosphorus component include, for example, phosphoric acid or its salts, and polyphosphoric acid or its salts. The starting materials usable for the component X include, for example, corresponding nitrates, sulfates, carbonates, phosphates, organic acid salts, halides, hydroxides and oxides of the elements X, that is copper, silver, zinc, cadmium, germanium, tin, lead, bismuth, aluminium, magnesium, calcium, strontium, barium, titanium, zirconium, niobium, tantalum, tungsten, manganese, iron, cobalt, nickel, cerium, thorium, rhenium, chromium, boron, potassium, rubidium, and cesium.

The catalyst of the invention can be prepared, for example, by reacting the starting materials for the constituent elements in water or in an organic solvent under heating, evaporating the reaction product to dryness and, if necessary, calcining the dried product.

If the calcination is required, the calcination temperature is preferably in the range of 200—500°C and more preferably in the range of 250—430°C.

Though the catalyst of the invention shows high catalytic activity without being supported on any carriers, preferable effects, such as improvements in thermal stability and the catalyst life and increase in the yield of methacrolein or methacrylic acid can be expected by supporting it on a suitable carrier.

Preferred carriers include silicon carbide, $\alpha$-alumina, aluminium powder, diatomaceous earth, titanium oxide and the like.

From the point of view of the structure of the catalyst, the preferred catalyst in the invention is one which has heteropoly-acid structure. The basic structure of the catalyst is phosphovanadomolybdic acid and other constituent elements are considered to contribute to the improvements in the catalytic activity and selectivity as well as in the stability of the structure by partially replacing the constituent elements in the phosphovanadomolybdic acid and being incorporated into the structure of the heteropoly-acid. The catalyst having such heteropoly-acid structure have very long catalyst-life. The heteropoly-acid structure can be identified by the characteristic X-ray diffraction peaks observed at $2\theta=8.0°$, 8.9°, 9.3° and the like. The presence of large amount of ammonium ions in the heteropoly-acid structure is not favorable, since such catalyst has not a long life. In order to prepare catalysts having very long life, the number of ammonium ions in the catalyst having the heteropoly-acid structure should preferably be limited to below 1, most preferably below 0.5, per 10 molybdenum atoms.

The catalyst having the heteropoly-acid structure can be readily prepared by general methods for preparing heteropoly-acids. For example, the catalyst having the heteropoly-acid structure can be prepared by reacting the starting materials for the constituent elements in water or in an organic solvent, and evaporating to dryness.

In the oxidation reaction according to the process of the invention, isobutylene and/or tertiary butanol and molecular oxygen or a molecular oxygen-containing gas are used as reactants. The molar ratio of the molecular oxygen to isobutylene and/or tertiary phenol is preferably in the range of 0.5 to 20 more preferably 1 to 10. In order to make the reaction to proceed smoothly, it is preferred to add water vapor to the feed gas in a molar ratio to the isobutylene and/or tertiary butanol of 1 to 20. The addition of water promotes the desorption of the products, methacrolein and methacrylic acid, from the catalyst surface and control the temperature of the catalyst bed.

The feed gas to be supplied may further contain other inert gases such as, for example, nitrogen, carbon dioxide, saturated hydrocarbons and the like.

The reaction of the invention may be conducted at a temperature of 250°C to 400°C, more preferably 280°C to 380°C and at a space velocity (SV) between 100 $hr^{-1}$ and 6000 $hr^{-1}$, more preferably 400 $hr^{-1}$ and 3000 $hr^{-1}$ based on the NTP standard. Since the increase in the space velocity has no substantial effect on the results of the reaction where the catalyst of this invention is employed, the reaction can be conducted at a high space velocity.

While the catalytic reaction can be carried out at a pressure either above or below the atmospheric pressure, it is suitably carried out generally at a pressure near the atmospheric pressure. The preferred pressure for the reaction in this invention lies between 1,01 and 5,07 bar.

The reaction of this invention can be conducted in any desired type of reactor such as a fixed bed, a fluidized bed or a moving bed reactor. In the following examples, the number of oxygen atoms in the catalyst compositions are not represented since the number of oxygen atoms is determined in accordance with the atomic ratio and valency of the other constituent elements.

The conversion, the yield of methacrolein, the yield of methacrylic acid and the selectivity to both methacrolein and methacrylic acid are defined as follows:

$$\text{Conversion (\%)} = \frac{\text{moles of isobutylene reacted + moles of tertiary butanol reacted}}{\text{moles of isobutylene fed + moles of tertiary butanol fed}} \times 100$$

$$\text{Yield of methacrolein (\%)} = \frac{\text{moles of methacrolein produced}}{\text{moles of isobutylene fed + moles of tertiary butanol fed}} \times 100$$

$$\text{Yield of methacrylic acid (\%)} = \frac{\text{moles of methacrylic acid produced}}{\text{moles of isobutylene fed + moles of tertiary butanol fed}} \times 100$$

Selectivity to (methacrolein + methacrylic acid) (%)

$$= \frac{\text{yield of methacrolein} + \text{yield of methacrylic acid}}{\text{Conversion}} \times 100$$

### Example A1

100 g of molybdenum trioxide, 6.3 g of vanadium pentoxide, 1.1 g of copper oxide, and 8.0 g of orthophosphoric acid were dispersed or dissolved in 1000 ml of deionized water. The resultant mixture was boiled under reflux with stirring for about 6 hours to obtain a clear orange red solution. After removing a slight amount of insoluble contents, it was evaporated to dryness on a hot bath. The dried product (catalyst) thus obtained had a composition: $Mo_{10}V_1P_1Cu_{0.2}$. The catalyst were confirmed to be heteropoly-acid by the observation of X-ray diffraction peaks at $2\theta$=8.0°, 8.9° and 9.3° and the like.

The catalyst was ground to 24 to 28 mesh and then charged into a tubular reactor made of Pyrex® glass of 18 mm inside diameter and the reactor was immersed in a fluidized bath. The feed gas of a composition wherein isobutylene:oxygen:nitrogen:water vapor = 1:4:18:6 (in molar ratio) was passed through the reactor at SV of 1000 $hr^{-1}$ (NTP standard) and subjected to oxidation reaction at a reaction temperature of 345°C. The following results were obtained:

| | |
|---|---|
| Conversion | 98.5% |
| Yield of methacrolein | 8.8% |
| Yield of methacrylic acid | 61.2% |
| Selectivity to methacrolein and methacrylic acid | 71.1% |

### Example A2

Example A1 was repeated except that tertiary butanol was used instead of isobutylene. The following results were obtained:

| | |
|---|---|
| Conversion | 100 % |
| Yield of methacrolein | 10.3% |
| Yield of methacrylic acid | 60.3% |

### Example A3—A33

1.1 g of copper oxide in Example A1 was replaced in each of the examples with 3.2 g of silver oxide, 1.1 g of zinc oxide, 2.0 g of cadmium oxide, 0.72 g of aluminum oxide, 1.4 g of germanium oxide, 2.1 g of tin oxide, 3.2 g of trilead tetraoxide, 3.2 g of bismuth oxide, 0.56 g of magnesium oxide, 0.78 g of calcium oxide, 1.4 g of strontium oxide, 2.1 g of barium oxide, 1.1 g of titanium oxide, 1.7 g of zirconium oxide, 1.8 g of niobium oxide, 3.0 g of tantalum oxide, 3.2 g of tungsten trioxide, 0.99 g of manganese oxide, 1.1 g of iron oxide, 1.1 g of tricobalt tetraoxide, 1.0 g of nickel oxide, 2.4 g of cerium oxide, 3.7 g of thorium oxide, 3.4 g of rhenium heptoxide, 1.4 g of chromium trioxide, 0.8 g of boric acid, 0.78 g of potassium hydroxide, 1.4 g of rubidium hydroxide and 2.1 g of cesium hydroxide, respectively, and dried products (catalysts) having compositions as shown in Table A1 were obtained. Then, the oxidation reactions were carried out using the above catalysts under the same conditions as in Example A1. The results are shown in Table A—1.

All of the dried products (catalysts) were confirmed to be heteropoly-acid by the observation of diffraction peaks at $2\theta$=8.0°, 8.9°, 9.3° and the like through X-ray diffraction analysis.

TABLE A–1

| Example No. | Composition of Catalyst | Reaction temperature (°C) | Conversion (%) | Yield of methacrolein (%) | Yield of methacrylic acid (%) | Selectivity to methacrolein + methacrylic acid (%) |
|---|---|---|---|---|---|---|
| A3 | $Mo_{10}V_1P_1Ag_{0.2}$ | 355 | 97.5 | 12.1 | 55.0 | 68.8 |
| A5 | $Mo_{10}V_1P_1Zn_{0.2}$ | 350 | 97.7 | 8.8 | 58.5 | 68.9 |
| A6 | $Mo_{10}V_1P_1Cd_{0.2}$ | 355 | 98.8 | 6.5 | 55.7 | 63.0 |
| A7 | $Mo_{10}V_1P_1Al_{0.2}$ | 348 | 98.0 | 9.5 | 58.8 | 69.7 |
| A8 | $Mo_{10}V_1P_1Ge_{0.2}$ | 350 | 99.0 | 10.0 | 59.5 | 70.2 |
| A9 | $Mo_{10}V_1P_1Sn_{0.2}$ | 350 | 97.8 | 6.7 | 57.7 | 65.8 |
| A10 | $Mo_{10}V_1P_1Pb_{0.2}$ | 350 | 97.8 | 10.0 | 55.1 | 66.7 |
| A12 | $Mo_{10}V_1P_1Bi_{0.2}$ | 350 | 98.5 | 9.3 | 58.7 | 69.0 |
| A13 | $Mo_{10}V_1P_1Mg_{0.2}$ | 355 | 93.8 | 21.3 | 42.0 | 67.5 |
| A14 | $Mo_{10}V_1P_1Ca_{0.2}$ | 355 | 95.0 | 20.5 | 40.0 | 63.7 |
| A15 | $Mo_{10}V_1P_1Sr_{0.2}$ | 350 | 97.3 | 8.8 | 55.0 | 65.6 |
| A16 | $Mo_{10}V_1P_1Ba_{0.2}$ | 350 | 96.8 | 10.1 | 52.1 | 64.3 |
| A17 | $Mo_{10}V_1P_1Ti_{0.2}$ | 350 | 98.5 | 9.5 | 57.1 | 67.6 |
| A18 | $Mo_{10}V_1P_1Zr_{0.2}$ | 350 | 98.7 | 11.0 | 58.8 | 70.7 |
| A19 | $Mo_{10}V_1P_1Nb_{0.2}$ | 350 | 95.0 | 21.0 | 48.2 | 72.8 |
| A20 | $Mo_{10}V_1P_1Ta_{0.2}$ | 355 | 96.3 | 15.2 | 53.8 | 71.7 |
| A21 | $Mo_{10}V_1P_1W_{0.2}$ | 350 | 98.9 | 7.8 | 60.1 | 68.7 |
| A22 | $Mo_{10}V_1P_1Mn_{0.2}$ | 350 | 94.8 | 25.0 | 40.2 | 68.8 |
| A23 | $Mo_{10}V_1P_1Fe_{0.2}$ | 350 | 99.5 | 5.1 | 60.5 | 65.9 |
| A24 | $Mo_{10}V_1P_1Co_{0.2}$ | 350 | 98.1 | 11.0 | 56.8 | 69.1 |
| A25 | $Mo_{10}V_1P_1Ni_{0.2}$ | 350 | 96.8 | 15.2 | 55.0 | 72.5 |
| A26 | $Mo_{10}V_1P_1Ce_{0.2}$ | 355 | 97.8 | 8.8 | 58.0 | 68.3 |
| A27 | $Mo_{10}V_1P_1Th_{0.2}$ | 355 | 97.1 | 8.5 | 57.2 | 67.7 |
| A28 | $Mo_{10}V_1P_1Re_{0.2}$ | 350 | 96.5 | 17.8 | 52.1 | 72.4 |
| A29 | $Mo_{10}V_1P_1Cr_{0.2}$ | 350 | 97.1 | 9.8 | 55.1 | 66.8 |
| A30 | $Mo_{10}V_1P_1B_{0.2}$ | 350 | 96.6 | 9.3 | 53.1 | 64.6 |
| A31 | $Mo_{10}V_1P_1K_{0.2}$ | 355 | 93.5 | 29.9 | 40.1 | 74.9 |
| A32 | $Mo_{10}V_1P_1Rb_{0.2}$ | 355 | 95.8 | 35.0 | 37.0 | 75.2 |
| A33 | $Mo_{10}V_1P_1Cs_{0.2}$ | 355 | 94.7 | 31.0 | 37.0 | 71.8 |

Example A34—A38

By repeating the procedure as described in Example A1 a number of the dried products having the composition shown in Table A—2 were obtained. Each dried product (catalyst) was used for the oxidation reaction in the same manner as in Example A1. The results are shown in Table A—2. These dried products were confirmed to be heteropoly-acid by the X-ray diffraction analysis.

Example A39—A43

In a similar manner as in Example A1 a few dried products having the compositions shown in Table A—3 were obtained. The oxidation reactions were carried out under the same reaction conditions as in Example A1 by the use of these dried products with the results shown in Table A—3. These dried products were confirmed to have heteropoly-acid structures by the X-ray diffraction analysis.

Examples A44—A45

With use of the catalyst of Example A1 the oxidation reactions were carried out in a similar manner as in Example A1 but at a different space velocity (SV) indicated in Table A—4. The results are shown in Table A—4. These results show that the increase in space velocity (SV) has no substantial effect on the results of the reaction.

TABLE A–2

| Example No. | Composition of catalyst | Reaction temperature (°C) | Conversion (%) | Yield of methacrolein (%) | Yield of methacrylic acid (%) | Selectivity to methacrolein + methacrylic acid (%) |
|---|---|---|---|---|---|---|
| A34 | $Mo_{10}V_1P_1Fe_{0.2}Sn_{0.1}$ | 350 | 99.5 | 9.8 | 60.8 | 71.0 |
| A35 | $Mo_{10}V_1P_1Ge_{0.2}Ce_{0.2}$ | 350 | 99.5 | 9.1 | 60.3 | 69.7 |
| A36 | $Mo_{10}V_1P_1W_{0.2}Fe_{0.2}$ | 350 | 98.8 | 10.0 | 58.5 | 69.3 |
| A37 | $Mo_{10}V_1P_1Al_{0.1}Th_{0.1}$ | 350 | 98.5 | 9.0 | 59.1 | 69.1 |
| A38 | $Mo_{10}V_1P_1Bi_{0.2}Co_{0.2}$ | 350 | 99.5 | 10.8 | 57.3 | 68.4 |

TABLE A–3

| Example No. | Composition of catalyst | Reaction temperature (°C) | Conversion (%) | Yield of methacrolein (%) | Yield of methacrylic acid (%) | Selectivity to methacrolein + methacrylic acid (%) |
|---|---|---|---|---|---|---|
| A39 | $Mo_{10}V_2P_1Cu_{0.02}$ | 360 | 97.0 | 15.0 | 51.1 | 68.1 |
| A40 | $Mo_{10}V_1P_3Cu_{0.1}$ | 350 | 98.5 | 6.2 | 59.1 | 66.3 |
| A41 | $Mo_{10}V_{0.5}P_{1.5}Cu_{0.3}$ | 340 | 98.8 | 8.5 | 55.0 | 64.3 |
| A42 | $Mo_{10}V_1P_1Cu_1$ | 340 | 100 | 3.3 | 60.3 | 63.6 |
| A43 | $Mo_{10}V_3P_1Cu_{0.5}$ | 360 | 97.5 | 11.8 | 53.0 | 66.5 |

TABLE A–4

| Example No. | Composition of catalyst | SV ($hr^{-1}$) | Reaction temperature (°C) | Conversion (%) | Yield of methacrolein (%) | Yield of methacrylic acid (%) | Selectivity to methacrolein + methacrylic acid (%) |
|---|---|---|---|---|---|---|---|
| A44 | $Mo_{10}V_1P_1Cu_{0.2}$ | 500 | 325 | 100 | 9.5 | 65.0 | 74.5 |
| A45 | $Mo_{10}V_1P_1Cu_{0.2}$ | 3000 | 360 | 98.1 | 11.5 | 58.5 | 66.2 |

Examples A46–A75:

With use of the catalysts of Examples A3 – A33 the oxidation reactions were carried out under the same reaction conditions as in Example A2. The results are shown in Table A–5 below.

TABLE A–5

| Example No. | Composition of catalyst | Reaction temperature (°C) | Conversion (%) | Yield of methacrolein (%) | Yield of methacrylic acid (%) |
|---|---|---|---|---|---|
| A46 | $Mo_{10}V_1P_1Ag_{0.2}$ | 355 | 100 | 12.5 | 56.2 |
| A47 | $Mo_{10}V_1P_1Zn_{0.2}$ | 350 | 100 | 9.3 | 59.0 |
| A48 | $Mo_{10}V_1P_1Cd_{0.2}$ | 355 | 100 | 7.3 | 55.9 |
| A49 | $Mo_{10}V_1P_1Al_{0.2}$ | 348 | 100 | 10.3 | 59.2 |
| A50 | $Mo_{10}V_1P_1Ge_{0.2}$ | 350 | 100 | 10.5 | 60.7 |
| A51 | $Mo_{10}V_1P_1Sn_{0.2}$ | 350 | 100 | 7.2 | 58.3 |
| A52 | $Mo_{10}V_1P_1Pb_{0.2}$ | 350 | 100 | 11.2 | 56.3 |
| A53 | $Mo_{10}V_1P_1Bi_{0.2}$ | 350 | 100 | 9.5 | 59.3 |
| A55 | $Mo_{10}V_1P_1Mg_{0.2}$ | 355 | 100 | 25.4 | 39.6 |
| A56 | $Mo_{10}V_1P_1Ca_{0.2}$ | 355 | 100 | 24.8 | 38.5 |
| A57 | $Mo_{10}V_1P_1Sr_{0.2}$ | 350 | 100 | 9.3 | 56.0 |
| A58 | $Mo_{10}V_1P_1Ba_{0.2}$ | 350 | 100 | 11.1 | 53.2 |
| A59 | $Mo_{10}V_1P_1Ti_{0.2}$ | 350 | 100 | 10.5 | 58.2 |
| A60 | $Mo_{10}V_1P_1Zr_{0.2}$ | 350 | 100 | 11.2 | 59.3 |
| A61 | $Mo_{10}V_1P_1Nb_{0.2}$ | 350 | 100 | 21.3 | 49.1 |
| A62 | $Mo_{10}V_1P_1Ta_{0.2}$ | 355 | 100 | 15.3 | 53.9 |
| A63 | $Mo_{10}V_1P_1W_{0.2}$ | 350 | 100 | 7.9 | 61.5 |
| A64 | $Mo_{10}V_1P_1Mn_{0.2}$ | 350 | 100 | 26.0 | 41.1 |
| A65 | $Mo_{10}V_1P_1Fe_{0.2}$ | 350 | 100 | 5.3 | 61.7 |
| A66 | $Mo_{10}V_1P_1Co_{0.2}$ | 350 | 100 | 11.2 | 56.9 |
| A67 | $Mo_{10}V_1P_1Ni_{0.2}$ | 350 | 100 | 15.3 | 56.2 |
| A68 | $Mo_{10}V_1P_1Ce_{0.2}$ | 355 | 100 | 8.9 | 59.2 |
| A69 | $Mo_{10}V_1P_1Th_{0.2}$ | 355 | 100 | 8.9 | 58.4 |
| A70 | $Mo_{10}V_1P_1Re_{0.2}$ | 350 | 100 | 17.9 | 52.3 |
| A71 | $Mo_{10}V_1P_1Cr_{0.2}$ | 350 | 100 | 9.9 | 55.9 |

TABLE A–5 (Continued)

| Example No. | Composition of catalyst | Reaction temperature (°C) | Conversion (%) | Yield of methacrolein (%) | Yield of methacrylic acid (%) |
|---|---|---|---|---|---|
| A72 | $Mo_{10}V_1P_1B_{0.2}$ | 350 | 100 | 9.9 | 54.3 |
| A73 | $Mo_{10}V_1P_1K_{0.2}$ | 355 | 100 | 30.5 | 40.8 |
| A74 | $Mo_{10}V_1P_1Rb_{0.2}$ | 355 | 100 | 35.8 | 37.9 |
| A75 | $Mo_{10}V_1P_1Cs_{0.2}$ | 355 | 100 | 31.7 | 38.1 |

Examples A76—A80

With use of the catalysts of Examples A34—A38 the oxidation reactions were carried out under the same reaction conditions as in Example A2. The results are shown in Table A—6.

Examples A81—A85

With use of the catalysts of Examples A39—A43 the oxidation reactions were carried out under the same reaction conditions as in Example A2. The results are shown in Table A—7.

Examples A86—A87

With use of the catalyst of Example A1 the oxidation reactions were carried out in a similar manner as in Example A2 except that the reaction was carried out at a space velocity indicated in Table A—8. The results are shown in Table A—8.

TABLE A–6

| Example No. | Composition of catalyst | Reaction temperature (°C) | Conversion (%) | Yield of methacrolein (%) | Yield of methacrylic acid (%) |
|---|---|---|---|---|---|
| A76 | $Mo_{10}V_1P_1Fe_{0.2}Sn_{0.1}$ | 350 | 100 | 10.2 | 61.2 |
| A77 | $Mo_{10}V_1P_1Ge_{0.2}Ce_{0.2}$ | 350 | 100 | 9.3 | 60.4 |
| A78 | $Mo_{10}V_1P_1W_{0.2}Fe_{0.2}$ | 350 | 100 | 10.5 | 59.0 |
| A79 | $Mo_{10}V_1P_1Al_{0.1}Th_{0.1}$ | 350 | 100 | 9.5 | 60.1 |
| A80 | $Mo_{10}V_1P_1Bi_{0.2}Co_{0.2}$ | 350 | 100 | 11.1 | 57.5 |

TABLE A–7

| Example No. | Composition of catalyst | Reaction temperature (°C) | Conversion (%) | Yield of methacrolein (%) | Yield of methacrylic acid (%) |
|---|---|---|---|---|---|
| A81 | $Mo_{10}V_2P_1Cu_{0.02}$ | 360 | 100 | 15.3 | 52.1 |
| A82 | $Mo_{10}V_1P_3Cu_{0.1}$ | 350 | 100 | 6.5 | 60.1 |
| A83 | $Mo_{10}V_{0.5}P_{1.5}Cu_{0.3}$ | 340 | 100 | 8.7 | 56.0 |
| A84 | $Mo_{10}V_1P_1Cu_1$ | 340 | 100 | 5.4 | 61.5 |
| A85 | $Mo_{10}V_3P_1Cu_{0.5}$ | 360 | 100 | 12.3 | 54.5 |

TABLE A–8

| Example No. | Composition of catalyst | SV ($hr^{-1}$) | Reaction temperature (°C) | Conversion (%) | Yield of methacrolein (%) | Yield of methacrylic acid (%) |
|---|---|---|---|---|---|---|
| A86 | $Mo_{10}V_1P_1Cu_{0.2}$ | 500 | 325 | 100 | 10.0 | 65.4 |
| A87 | $Mo_{10}V_1P_1Cu_{0.2}$ | 3000 | 360 | 100 | 11.7 | 59.0 |

Example A88

The same catalyst as obtained in Example A1 was used and a feed gas having such a composition that isobutylene:tertiary butanol:oxygen:nitrogen:water vapor = 1:1:8:36:12 was passed through the reactor at a SV of 1000 $hr^{-1}$ (NTP standard) at a temperature of 350°C. The reaction results were as follows:

| | |
|---|---|
| Conversion | 98% |
| Yield of methacrolein | 10.5% |
| Yield of methacrylic acid | 60.8% |
| Selectivity to methacrolein and methacrylic acid | 72.8% |

Example A89

The reactions of Example A1, A2, A3—A33, and A46—A75 were continued for 30 days. It was found that no substantial changes in reaction results were recognized. The reaction results obtained after the lapse of 30 days are shown in Tables A—9 and A—10.

TABLE A–9

| Example No. for continuous reaction | Reaction time (days) | Conversion (%) | Yield of methacrolein (%) | Yield of methacrylic acid (%) |
|---|---|---|---|---|
| A1 | 30 | 98.6 | 8.8 | 61.3 |
| A3 | 30 | 96.8 | 12.7 | 54.8 |
| A5 | 30 | 97.9 | 8.7 | 58.7 |
| A6 | 30 | 98.6 | 6.8 | 55.4 |
| A7 | 30 | 98.2 | 9.3 | 59.0 |
| A8 | 30 | 99.1 | 9.8 | 60.0 |
| A9 | 30 | 97.8 | 6.9 | 57.8 |
| A10 | 30 | 97.9 | 9.8 | 55.3 |
| A12 | 30 | 98.6 | 9.3 | 58.9 |
| A13 | 30 | 93.0 | 21.5 | 41.8 |
| A14 | 30 | 94.3 | 20.6 | 40.0 |
| A15 | 30 | 96.9 | 8.8 | 54.7 |
| A16 | 30 | 96.5 | 10.2 | 51.9 |
| A17 | 30 | 98.6 | 9.4 | 57.3 |
| A18 | 30 | 98.7 | 10.9 | 58.8 |
| A19 | 30 | 95.0 | 21.3 | 48.1 |
| A20 | 30 | 96.2 | 15.5 | 53.5 |
| A21 | 30 | 98.9 | 7.7 | 60.2 |
| A22 | 30 | 94.9 | 25.2 | 40.1 |
| A23 | 30 | 99.5 | 5.0 | 60.8 |
| A24 | 30 | 98.3 | 10.8 | 56.9 |
| A25 | 30 | 97.0 | 15.0 | 55.2 |
| A26 | 30 | 97.6 | 9.0 | 57.8 |
| A27 | 30 | 96.8 | 8.7 | 57.0 |
| A28 | 30 | 96.6 | 17.6 | 52.4 |
| A29 | 30 | 97.4 | 9.7 | 55.6 |
| A30 | 30 | 96.5 | 9.5 | 52.8 |
| A31 | 30 | 92.8 | 30.3 | 39.8 |
| A32 | 30 | 95.3 | 36.1 | 36.5 |
| A33 | 30 | 94.0 | 31.9 | 36.6 |

TABLE A–10

| Example No. for continuous reaction | Reaction time (days) | Conversion (%) | Yield of methacrolein (%) | Yield of methacrylic acid (%) |
|---|---|---|---|---|
| A2 | 30 | 100 | 10.1 | 60.4 |
| A46 | 30 | 100 | 12.9 | 56.0 |
| A47 | 30 | 100 | 9.2 | 59.1 |
| A48 | 30 | 100 | 7.2 | 55.8 |
| A49 | 30 | 100 | 10.2 | 59.4 |
| A50 | 30 | 100 | 10.3 | 60.9 |
| A51 | 30 | 100 | 7.2 | 58.4 |
| A52 | 30 | 100 | 11.4 | 56.1 |
| A54 | 30 | 100 | 9.4 | 59.5 |
| A55 | 30 | 100 | 25.8 | 39.4 |
| A56 | 30 | 100 | 25.0 | 38.3 |
| A57 | 30 | 100 | 9.2 | 56.1 |
| A58 | 30 | 100 | 11.3 | 53.1 |
| A59 | 30 | 100 | 10.4 | 58.3 |
| A60 | 30 | 100 | 11.0 | 59.5 |
| A61 | 30 | 100 | 21.3 | 49.2 |
| A62 | 30 | 100 | 15.6 | 53.7 |
| A63 | 30 | 100 | 7.9 | 61.6 |
| A64 | 30 | 100 | 26.0 | 41.0 |
| A65 | 30 | 100 | 5.3 | 61.9 |
| A66 | 30 | 100 | 11.3 | 57.0 |
| A67 | 30 | 100 | 15.3 | 56.4 |
| A68 | 30 | 100 | 9.3 | 59.1 |
| A69 | 30 | 100 | 9.2 | 58.3 |
| A70 | 30 | 100 | 17.9 | 52.4 |
| A71 | 30 | 100 | 9.8 | 56.0 |
| A72 | 30 | 100 | 9.9 | 54.2 |
| A73 | 30 | 100 | 31.3 | 40.6 |
| A74 | 30 | 100 | 36.4 | 37.8 |
| A75 | 30 | 100 | 32.5 | 37.9 |

Example B1

100 g of molybdenum trioxide, 6.3 g of vanadium pentoxide, 3.0 g of copper phosphate, 2.1 g of tin oxide, and 6.4 g of orthophosphoric acid were dispersed or dissolved in 1000 ml of deionized water. The resultant mixture was boiled under reflux with stirring for about 6 hours to obtain a clear orange red solution. After removing a slight amount of insoluble contents, it was evaporated to dryness on a hot water bath. The dried product (catalyst) thus obtained had a composition of $Mo_{10}V_1Cu_{0.3}Sn_{0.2}P_1$. This catalyst was confirmed to be heteropoly-acid by the observation of X-ray diffraction peaks at $2\theta=8.0°$, 8.9°, 9.3° and the like. The feed gas of a composition wherein isobutylene:oxygen:nitrogen:water vapor = 1:4:18:6 was passed through a reactor charged with the catalyst at a SV of 1000 $hr^{-1}$ (NTP standard) and at a reaction temperature of 350°C. The reaction results were as follows:

| | |
|---|---|
| Conversion | 99.2% |
| Yield of methacrolein | 8.7% |
| Yield of methacrylic acid | 62.1% |
| Selectivity to methacrolein and methacrylic acid | 71.4% |

Example B2

The catalyst obtained in Example B1 was used and a feed gas having such a composition that tertiary butanol:oxygen:nitrogen:water vapor = 1:4:18:6 was passed through a reactor charged with the catalyst at a SV of 1000 $hr^{-1}$ and at a temperature of 345°C. The reaction results were as follows:

| | |
|---|---|
| Conversion | 100% |
| Yield of methacrolein | 8.9% |
| Yield of methacrylic acid | 62.9% |
| Selectivity to methacrolein and methacrylic acid | 71.8% |

Examples B3—B18

2.1 g of tin oxide in Example B1 was replaced in each of the examples with 3.7 g of thorium oxide, 0.72 g of aluminium oxide, 1.4 g of germanium oxide, 1.0 g of nickel oxide, 1.1 g of iron oxide, 1.1 g of tricobalt tetroxide, 0.39 g of potassium hydroxide, 0.7 g of rubidium hydroxide, 1.1 g of cesium hydroxide, 1.1 g of zinc oxide, 1.1 g of titanium oxide, 3.2 g of trilead tetroxide, 3.4 g of rhenium heptoxide, 1.7 g of zirconium oxide, 1.4 g of chromium trioxide, and 3.2 g of bismuth oxide and dried products (catalysts) having compositions indicated in Table B—1 were obtained. Then the oxidation reactions were carried out using the above catalysts under the same conditions as in Example B1. The reaction results are shown in Table B—1.

All of the dried products were confirmed to be a heteropoly-acid by the observation of X-ray diffraction peaks at $2\theta=8.0°$, 8.9°, 9.3° and the like.

TABLE B–1

| Example No. | Composition of catalyst | Reaction temperature (°C) | Conversion (%) | Yield of methacrolein (%) | Yield of methacrylic acid (%) | Selectivity to methacrolein + methacrylic acid (%) |
|---|---|---|---|---|---|---|
| B3 | $Mo_{10}V_1Cu_{0.3}Th_{0.2}P_1$ | 355 | 99.6 | 8.4 | 62.8 | 71.5 |
| B4 | $Mo_{10}V_1Cu_{0.3}Al_{0.2}P_1$ | 345 | 99.1 | 8.6 | 63.5 | 72.8 |
| B5 | $Mo_{10}V_1Cu_{0.3}Ge_{0.2}P_1$ | 345 | 99.0 | 8.2 | 63.8 | 72.7 |
| B6 | $Mo_{10}V_1Cu_{0.3}Ni_{0.2}P_1$ | 350 | 99.3 | 8.5 | 62.4 | 71.4 |
| B7 | $Mo_{10}V_1Cu_{0.3}Fe_{0.2}P_1$ | 345 | 99.5 | 7.1 | 63.6 | 71.1 |
| B8 | $Mo_{10}V_1Cu_{0.3}Co_{0.2}P_1$ | 350 | 99.1 | 7.7 | 62.5 | 70.8 |
| B9 | $Mo_{10}V_1Cu_{0.3}K_{0.1}P_1$ | 360 | 99.7 | 8.7 | 62.4 | 71.3 |
| B10 | $Mo_{10}V_1Cu_{0.3}Rb_{0.1}P_1$ | 360 | 99.4 | 8.4 | 62.3 | 71.1 |
| B11 | $Mo_{10}V_1Cu_{0.3}Cs_{0.1}P_1$ | 360 | 99.2 | 8.6 | 62.5 | 71.7 |
| B12 | $M_{10}V_1Cu_{0.3}Zn_{0.2}P_1$ | 350 | 99.3 | 8.2 | 62.8 | 71.5 |
| B13 | $M_{10}V_1Cu_{0.3}Ti_{0.2}P_1$ | 350 | 99.5 | 8.2 | 62.6 | 71.2 |
| B14 | $M_{10}V_1Cu_{0.3}Pb_{0.2}P_1$ | 350 | 99.6 | 8.6 | 62.5 | 71.4 |
| B15 | $Mo_{10}V_1Cu_{0.3}Re_{0.2}P_1$ | 350 | 99.3 | 8.7 | 62.9 | 72.1 |
| B16 | $M_{10}V_1Cu_{0.3}Zr_{0.2}P_1$ | 350 | 99.8 | 8.3 | 62.5 | 70.9 |
| B17 | $Mo_{10}V_1Cu_{0.3}Cr_{0.2}P_1$ | 350 | 98.5 | 8.6 | 62.7 | 72.4 |
| B18 | $Mo_{10}V_1Cu_{0.3}Bi_{0.2}P_1$ | 350 | 99.5 | 8.6 | 63.1 | 72.1 |

Examples B19 – B22:

The dried products of the compositions indicated in Table B–2 were obtained in the same manner as in Example B1 and used to carry out the oxidation reaction in the same manner as in Example B1. The results are shown in Table B–2.

TABLE B–2

| Example No. | Composition of catalyst | Reaction temperature (°C) | Conversion (%) | Yield of methacrolein (%) | Yield of methacrylic acid (%) | Selectivity to methacrolein + methacrylic acid (%) |
|---|---|---|---|---|---|---|
| B19 | $Mo_{10}V_1Cu_{0.2}Th_{0.1}Sn_{0.1}P_1$ | 350 | 99.1 | 9.9 | 62.8 | 73.4 |
| B20 | $Mo_{10}V_1Cu_{0.2}Ge_{0.1}Ni_{0.1}P_1$ | 345 | 99.2 | 10.8 | 61.5 | 72.9 |
| B21 | $Mo_{10}V_1Cu_{0.2}Rb_{0.1}Sn_{0.1}P_1$ | 350 | 99.5 | 12.5 | 62.2 | 75.1 |
| B22 | $Mo_{10}V_1Cu_{0.2}Zr_{0.1}Th_{0.1}P_1$ | 350 | 99.7 | 10.7 | 61.9 | 72.8 |

Examples B23 – B27:

The dried products of the compositions indicated in Table B3 were obtained in the same manner as in Example B1 and used to carry out the oxidation reaction under the same conditions as in Example B1. The results are shown in Table B–3 below.

TABLE B–3

| Example No. | Composition of catalyst | Reaction temperature (°C) | Conversion (%) | Yield of methacrolein (%) | Yield of methacrylic acid (%) | Selectivity to methacrolein + methacrylic acid (%) |
|---|---|---|---|---|---|---|
| B23 | $Mo_{10}V_2Cu_{0.3}Sn_{0.1}P_1$ | 360 | 99.2 | 8.7 | 56.6 | 65.8 |
| B24 | $Mo_{10}V_1Cu_{0.2}Sn_{0.1}P_2$ | 350 | 99.1 | 8.6 | 61.2 | 70.4 |
| B25 | $Mo_{10}V_1Cu_{1.0}Sn_{0.2}P_1$ | 340 | 99.8 | 5.7 | 62.1 | 67.9 |
| B26 | $Mo_{10}V_1Cu_{0.05}Ni_{0.05}P_1$ | 350 | 99.8 | 8.7 | 62.3 | 71.1 |
| B27 | $Mo_{10}V_1Cu_{0.1}Sn_{0.03}P_1$ | 350 | 99.8 | 5.9 | 62.4 | 68.4 |

Examples B28 – B29:

The catalyst of Example B1 was used to carry out the oxidation reaction under the same reaction conditions as in Example B1 using space velocities (SV) indicated in Table B–4. The reaction results are shown in Table B–4. These results show that the increase in the space velocity (SV) has no substantial effect on the results of the reaction.

TABLE B–4

| Example No. | Composition of catalyst | SV ($hr^{-1}$) | Reaction temperature (°C) | Conversion (%) | Yield of methacrolein (%) | Yield of methacrylic acid (%) | Selectivity to methacrolein + methacrylic acid (%) |
|---|---|---|---|---|---|---|---|
| B28 | $Mo_{10}V_1Cu_{0.3}Sn_{0.2}P_1$ | 500 | 325 | 100 | 7.0 | 66.0 | 73.0 |
| B29 | $Mo_{10}V_1Cu_{0.3}Sn_{0.2}P_1$ | 3000 | 360 | 99.5 | 10.5 | 60.1 | 70.6 |

Examples B30 – B45:

The catalysts obtained in Examples B3 – B18 were each used to carry out the oxidation reaction under conditions as in Example B2. The results are shown in Table B–5 below.

TABLE B–5

| Example No. | Composition of catalyst | Reaction temperature (°C) | Conversion (%) | Yield of methacrolein (%) | Yield of methacrylic acid (%) | Selectivity to methacrolein + methacrylic acid (%) |
|---|---|---|---|---|---|---|
| B30 | $Mo_{10}V_1Cu_{0.3}Th_{0.2}P_1$ | 355 | 100 | 8.8 | 61.8 | 70.6 |
| B31 | $Mo_{10}V_1Cu_{0.3}Al_{0.2}P_1$ | 345 | 100 | 9.0 | 62.0 | 71.0 |
| B32 | $Mo_{10}V_1Cu_{0.3}Ge_{0.2}P_1$ | 345 | 100 | 8.6 | 62.5 | 71.1 |
| B33 | $Mo_{10}V_1Cu_{0.3}Ni_{0.2}P_1$ | 350 | 100 | 8.9 | 61.8 | 70.7 |
| B34 | $Mo_{10}V_1Cu_{0.3}Fe_{0.2}P_1$ | 345 | 100 | 7.5 | 63.0 | 70.5 |
| B35 | $Mo_{10}V_1Cu_{0.3}Co_{0.2}P_1$ | 350 | 100 | 8.2 | 61.8 | 70.0 |
| B36 | $Mo_{10}V_1Cu_{0.3}K_{0.1}P_1$ | 360 | 100 | 9.2 | 61.7 | 70.9 |
| B37 | $Mo_{10}V_1Cu_{0.3}Rb_{0.1}P_1$ | 360 | 100 | 8.8 | 61.6 | 70.4 |
| B38 | $Mo_{10}V_1Cu_{0.3}Cs_{0.1}P_1$ | 360 | 100 | 9.0 | 61.8 | 70.8 |
| B39 | $Mo_{10}V_1Cu_{0.3}Zn_{0.2}P_1$ | 350 | 100 | 8.6 | 62.1 | 70.7 |
| B40 | $Mo_{10}V_1Cu_{0.3}Ti_{0.2}P_1$ | 350 | 100 | 8.6 | 62.0 | 70.6 |
| B41 | $Mo_{10}V_1Cu_{0.3}Pb_{0.2}P_1$ | 350 | 100 | 9.0 | 62.3 | 71.3 |
| B42 | $Mo_{10}V_1Cu_{0.3}Re_{0.2}P_1$ | 350 | 100 | 9.1 | 62.3 | 71.4 |
| B43 | $Mo_{10}V_1Cu_{0.3}Zr_{0.2}P_1$ | 350 | 100 | 8.7 | 62.0 | 70.7 |
| B44 | $Mo_{10}V_1Cu_{0.3}Cr_{0.2}P_1$ | 350 | 100 | 9.0 | 62.4 | 71.4 |
| B45 | $Mo_{10}V_1Cu_{0.3}Bi_{0.2}P_1$ | 350 | 100 | 9.0 | 62.8 | 71.8 |

Examples B46 – B49:

The catalysts obtained in Examples B19 – B22 were used to carry out the oxidation reaction under the same conditions as in Example B2. The results are shown in Table B–6.

TABLE B–6

| Example No. | Composition of catalyst | Reaction temp. (°C) | Con-version (%) | Yield of meth-acrolein (%) | Yield of meth-acrylic acid (%) | Selectivity to meth-acrolein + methacrylic acid (%) |
|---|---|---|---|---|---|---|
| B46 | $Mo_{10}V_1Cu_{0.2}Th_{0.1}Sn_{0.1}P_1$ | 350 | 100 | 10.3 | 63.5 | 73.8 |
| B47 | $Mo_{10}V_1Cu_{0.2}Ge_{0.1}Ni_{0.1}P_1$ | 345 | 100 | 11.2 | 62.2 | 73.4 |
| B48 | $Mo_{10}V_1Cu_{0.2}Rb_{0.1}Sn_{0.1}P_1$ | 350 | 100 | 12.7 | 63.5 | 76.2 |
| B49 | $Mo_{10}V_1Cu_{0.2}Zr_{0.1}Th_{0.1}P_1$ | 350 | 100 | 11.3 | 62.4 | 73.7 |

Examples B50 – B54:

The catalysts obtained in Examples B23 – B27 were used to carry out the oxidation reaction under the same conditions as in Example B2. The results are shown in Table B–7.

TABLE B–7

| Example No. | Composition of catalyst | Reaction temp. (°C) | Con-version (%) | Yield of meth-acrolein (%) | Yield of methacrylic acid (%) | Selectivity to meth-acrolein + methacrylic acid (%) |
|---|---|---|---|---|---|---|
| B50 | $Mo_{10}V_2Cu_{0.3}Sn_{0.1}P_1$ | 360 | 100 | 9.3 | 57.2 | 66.5 |
| B51 | $Mo_{10}V_1Cu_{0.2}Sn_{0.1}P_2$ | 350 | 100 | 9.3 | 61.9 | 71.2 |
| B52 | $Mo_{10}V_3Cu_{1.0}Sn_{0.2}P_1$ | 340 | 100 | 6.4 | 62.8 | 69.2 |
| B53 | $Mo_{10}V_1Cu_{0.05}Ni_{0.05}P_1$ | 350 | 100 | 8.9 | 62.5 | 71.4 |
| B54 | $Mo_{10}V_1Cu_{0.1}Sn_{0.03}P_1$ | 350 | 100 | 6.5 | 62.3 | 68.8 |

Examples B55 – B56:

The catalyst obtained in Example B1 was used to carry out the oxidation reaction under the same conditions as in Example B2 using different space velocities (SV) indicated in Table B–8. The results are shown in Table B–8 below.

TABLE B–8

| Example No. | Composition of catalyst | SV ($hr^{-1}$) | Reaction temp. (°C) | Con-version (%) | Yield of meth-acrolein (%) | Yield of meth-acrylic acid (%) | Selectivity to meth-acrolein + methacrylic acid (%) |
|---|---|---|---|---|---|---|---|
| B55 | $Mo_{10}V_1Cu_{0.3}Sn_{0.2}P_1$ | 500 | 325 | 100 | 8.7 | 66.5 | 75.2 |
| B56 | $Mo_{10}V_1Cu_{0.3}Sn_{0.2}P_1$ | 3000 | 360 | 100 | 9.5 | 61.1 | 70.6 |

Example B57

The catalyst obtained in Example B1 was used to carry out the oxidation reaction at a temperature of 350°C by passing a feed gas having such a composition that isobutylene:tertiary:butanol: oxygen:nitrogen:water vapor = 1:1:8:36:12 (NTP standard) at an SV of 1000 $hr^{-1}$ through a reactor charged with the catalyst. The reaction results were as follows:

| | |
|---|---|
| Conversion | 100% |
| Yield of methacrolein | 10.9% |
| Yield of methacrylic acid | 61.9% |
| Selectivity to methacrolein and methacrylic acid | 72.8% |

Example B58

The reactions of Examples B1—B18 and B30—B45 were each continued for 30 days but no substantial changes in results were recognized.

The reaction results obtained after the reaction time of 30 days are shown in Tables B—9 and B—10.

TABLE B–9

| Example No. for continuous reaction | Reaction time (days) | Conversion (%) | Yield of methacrolein (%) | Yield of methacrylic acid (%) |
|---|---|---|---|---|
| B1 | 30 | 99.3 | 8.7 | 62.2 |
| B3 | 30 | 99.5 | 8.5 | 62.8 |
| B4 | 30 | 99.2 | 8.6 | 63.7 |
| B5 | 30 | 99.3 | 8.1 | 63.9 |
| B6 | 30 | 99.4 | 8.5 | 62.5 |
| B7 | 30 | 99.8 | 6.9 | 63.8 |
| B8 | 30 | 99.3 | 7.5 | 62.8 |
| B9 | 30 | 99.6 | 8.8 | 62.4 |
| B10 | 30 | 99.3 | 8.5 | 62.2 |
| B11 | 30 | 99.1 | 8.8 | 62.3 |
| B12 | 30 | 99.5 | 8.1 | 62.9 |
| B13 | 30 | 99.6 | 8.2 | 62.7 |
| B14 | 30 | 99.6 | 8.5 | 62.6 |
| B15 | 30 | 99.5 | 8.7 | 63.0 |
| B16 | 30 | 100 | 8.1 | 62.6 |
| B17 | 30 | 98.6 | 8.6 | 62.7 |
| B18 | 30 | 99.6 | 8.6 | 63.1 |

TABLE B–10

| Example No. for continuous reaction | Reaction time (days) | Conversion (%) | Yield of methacrolein (%) | Yield of methacrylic acid (%) |
|---|---|---|---|---|
| B2 | 30 | 100 | 8.9 | 63.2 |
| B30 | 30 | 100 | 8.9 | 61.8 |
| B31 | 30 | 100 | 9.0 | 62.4 |
| B32 | 30 | 100 | 8.7 | 62.6 |
| B33 | 30 | 100 | 8.8 | 61.9 |
| B34 | 30 | 100 | 7.4 | 63.1 |
| B35 | 30 | 100 | 8.0 | 61.8 |
| B36 | 30 | 100 | 9.4 | 61.5 |
| B37 | 30 | 100 | 8.9 | 61.5 |
| B38 | 30 | 100 | 9.0 | 61.7 |
| B39 | 30 | 100 | 8.7 | 62.3 |
| B40 | 30 | 100 | 8.7 | 62.2 |
| B41 | 30 | 100 | 9.0 | 62.4 |
| B42 | 30 | 100 | 9.0 | 62.3 |
| B43 | 30 | 100 | 8.9 | 62.1 |
| B44 | 30 | 100 | 9.1 | 62.5 |
| B45 | 30 | 100 | 9.1 | 62.9 |

Example B59

100 g of ammonium molybdate was dissolved in 500 ml of deionized water, to which 4.5 g of ammonium metavanadate was added, followed by heating for dissolution at about 50°C. 10.8 g of ammonium phosphate, 16.75 g of copper phosphate and 4.7 g aluminum phosphate were added to the solution, which was then evaporated to dryness under continued heating and stirring. The resulting product was dried at 110°C for 24 hours. The dried product was ground to 6—9 mesh and charged in a quartz tube with an inner diameter of 25 mm and a length of 50 cm and calcined at 400°C for 6 hours under the supply of air at a rate of 10 l/hr. The obtained catalyst had a composition of $Mo_{10}V_{0.7}Cu_{0.7}Al_{0.7}P_{2.3}$.

This catalyst was used to carry out the oxidation reaction of isobutylene at a temperature of 345°C in the same manner as in Example B1. The reaction results were as follows:

| | |
|---|---|
| Conversion | 99.0% |
| Yield of methacrolein | 8.7% |
| Yield of methacrylic acid | 61.5% |
| Selectivity to methacrolein and methacrylic acid | 70.9% |

**Claims**

1. A process for producing methacrolein and methacrylic acid by vapor phase oxidation of isobutylene and/or tertiary butanol with molecular oxygen or a molecular oxygen-containing gas in the presence of a catalyst comprising phosphorus, vanadium and molybdenum, characterized in that the catalyst has the formula

$$Mo_aV_bP_cX_dO_e$$

wherein X represents at least one of the elements Cu, Ag, Zn, Cd, Al, Ge, Sn, Pb, Bi, Mg, Ca, Sr, Ba, Ti, Zr, Nb, Ta, W, Mn, Fe, Co, Ni, Ce, Th, Re, Cr, B, K, Rb or Cs, and a, b, c, d and e represent the atomic ratio of the elements where *a* is 10, *b* is a nuimber of 6 or less than 6 excluding O, *c* is a number of 0.5 to 10, *d* is a number of 0,01 to 5, and *e* is a number determined depending on the valency and atomic ratio of the other elements.

2. A process according to claim 1, characterized in that the catalyst has heteropolyacid structure.

3. A process according to claim 1, characterized in that *a* is 10, *b* is a number of 0.5 to 3, *c* is a number of 0.5 to 3, and *d* is a number of 0,01 to 2.

4. A process according to claim 3, characterized in that the catalyst has heteropolyacid structure.

5. A process according to claim 1, characterized in that the reaction temperature is between 250 and 400°C.

6. A process according to claim 1, characterized in that the reaction is conducted in the presence of water vapor.

7. A process according to claim 1, characterized in that the catalyst has the following formula:

$$Mo_{a'}\ V_{b'}\ P_{c'}\ Cu_{d'}\ X'_{e'}\ O_f$$

wherein X' represents at least one of the elements tin, thorium, aluminum, germanium, nickel, iron, cobalt, potassium, rubidium, cesium, zinc, titanium, lead, rhenium, zirconium, chromium or bismuth, and *a', b', c', d', e'* and *f'* represent the atomic ratio of the respective elements wherein *a'* is 10, *b'* is a number of 6 or less than 6 excluding O, *c'* is a number of 0.5 to 10, *d'* is a number of 3 or less than 3 excluding O,m *e'* is a number of 3 or less than 3 excluding O, and *f'* is a number determined depending on the valency and atomic ratio of the other elements, provided that *d'* + *e'* is a number of 0.01 to 5.

8. The process according to claim 7, characterized in that the catalyst has heteropolyacid structure.

9. The process according to claim 7, characterized in that *a'* is 10, *b'* is a number of 0.5 to 3, *c'* is a number of 0,5 to 3, *d'* is a number of 0,01 to 1,0, and *e'* is a number of 0.01 to 1,0.

10. The process according to claim 9, characterized in that the catalyst has heteropolyacid structure.

**Revendications**

1. Procédé de production de méthacroléine et d'acide méthacrylique par oxydation en phase vapeur d'isobutylène et/ou de tertio-butanol par de l'oxygène moléculaire ou un gaz contenant de l'oxygène moléculaire en présence d'un catalyseur comprenant phosphore, vanadium et molybdène, caractérisé en ce que le catalyseur présente la formule:

$$Mo_aV_bP_cX_dO_e$$

dans laquelle:

X est au moins un des éléments Cu, Ag, Zn, Cd, Al, Ge, Sn, Pb, Bi, Mg, Ca, Sr, Ba, Ti, Zr, Nb, Ta, W, Mn, Fe, Co, Ni, Ce, Th, Re, Cr, B, K, Rb ou Cs, et

a, b, c, d et e, sont des rapports atomiques des éléments, étant entendu que:

a est égal à 10,

b est un nombre égal à 6 ou inférieur à 6 mais différent de O.

c est un nombre compris entre 0,5 et 10,

d est une nombre compris entre 0,01 et 5,

e est un nombre choisi en fonction de la valence et des rapports atomiques des autres éléments présents.

2. Procédé selon la revendication 1, caractérisé en ce que le catalyseur présente une structure d'hétéropoly-acide.

3. Procédé selon la revendication 1, caractérisé en ce que:

a est égal à 10,

b est un nombre compris entre 0,5 et 3,

c est un nombre compris entre 0,5 et 3, et

d est un nombre compris entre 0,01 et 2.

4. Procédé selon la revendication 3, caractérisé en ce que le catalyseur présente une structure d'hétéropoly-acide.

5. Procédé selon la revendication 1, caractérisé en ce que la température réactionnelle est comprise entre 250 et 400°C.

6. Procédé selon la revendication 1, caractérisé en ce que l'réaction est effectuée en présence de vapeur d'eau.

7. Procédé selon la revendication 1, caractérisé en ce que le catalyseur présente la formule:

$$Mo_{a'} V_{b'} P_{c'} Cu_{d'} X_{e'} O_{f'}$$

dans laquelle:

X' représente au moins un des éléments consistant en étain, thorium, aluminium, germanium, nickel, fer, cobalt, potassium, rubidium, cesium, zinc, titane, plomb, rhénium, zirconium, chrome ou bistmuth. et a', b', c', d', e', et f', représentent les rapports atomiques des différents éléments, à savoir:

a' est égal à ,

b' est un nombre égal à 6 ou inférieur à 6, mais différent de O,

c' est une nombre compris entre 0,5 et 10,

d' est un nombre égal à 3 ou inférieur à 3, mais différent de O,

e' est un nombre égal à 3 ou inférieur à 3, mais différent de O, et

f' est un nombre déterminé en fonction de la valence et des rapports atomiques des différents éléments présents, étant entendu que: d + e' est égal à 0,01 à 5.

8. Procédé selon la revendication 7, caractérisé en ce que le catalyseur présente une structure d'hétéropoly-acide.

9. Procédé selon la revendication 7, caractérisé en ce que:

a' est égal à 10,

b' est un nombre compris entre 0,5 et 3,

c' est un nombre compris entre 0,5 et 3,

d' est un nombre compris entre 0,01 et 1,0, et

e' est un nombre compris entre 0,01 et 1,0.

10. Procédé selon la revendication 9, caractérisé en ce que le catalyseur présente une structure d'hétéropoly-acide.

**Patentansprüche**

1. Verfahren zur Herstellung von Methacrolein und Methacrylsäure durch Dampfphasenoxydation von Isobutylen und/oder tert.-Butanol mit molekularem Sauerstoff oder einem molekularen Sauerstoff enthaltenden Gas in Gegenwart eines Katalysators, der Phosphor, Vanadium und Molybdän enthält, dadurch gekennzeichnet, daß der Katalysator die Formel

$$Mo_aV_bP_cX_dO_e$$

hat, in der X mindestens eines der Elemente Cu, Ag, Zn, Cd, Al, Ge, Sn, Pb, Bi, Mg, Ca, Sr, Ba, Ti, Zr, Nb, Ta, W, Mn, Fe, Co, Ni, Ce, Th, Re, Cr, B, K, Rb oder Cs bedeutet, und a, b, c, d und 3 die Anzahl der Atome der einzelnen Elemente darstellen, wobei a = 10, b eine Zahl von 6 oder weniger, ausgenommen O, c eine Zahl von 0,5 bis 10, d eine Zahl von 0,01 bis 5 und e eine Zahl ist, die von der Wertigkeit und der Anzahl der Atome der anderen Elemente bestimmt wird.

2. Verfahren nach Anspruch 1, dadurch gekennzeichnet, daß der Katalysator Heteropolysäurestruktur hat.

3. Verfahren nach Anspruch 1, dadurch gekennzeichnet, daß a = 10, b ein Zahl von 0,5 bis 3, c ein Zahl von 0,5 bis 3 und d eine Zahl von 0,01 bis 2 ist.

4. Verfahren nach Anspruch 3, dadurch gekennzeichnet, daß der Katalysator Heteropolysäurestruktür hat.

5. Verfahren nach Anspruch 1, dadurch gekennzeichnet, daß die Reaktionstemperatur zwischen 250 und 400°C liegt.

6. Verfahren nach Anspruch 1, dadurch gekennzeichnet, daß die Umsetzung in Gegenwart von Wasserdampf durchgeführt wird.

7. Verfahren nach Anspruch 1, dadurch gekennzeichnet, daß der Katalysator die Formel

$$Mo_{a'}\ V_{b'}\ P_{c'}\ Cu_{d'}\ X'_{e'}\ O_{f}$$

hat, in der X' mindestens eines der Elemente Zinn, Thorium, Aluminium, Germanium, Nickel, Eisen, Kobalt, Kalium, Rubidium, Cäsium, Zink, Titan, Blei, Rhenium, Zirkon, Chrom oder Wismut ist, und a', b', c', d', e' und f' die jeweilige Anzahl der Atome der Elemente bedeuten, wobei a' = 10, b' eine Zahl von 6 oder weniger, ausgenommen O, c' eine Zahl von 0,5 bis 10, d' eine Zahl von 3 oder weniger, ausgenommen O, e' eine Zahl von 3 oder weniger, ausgenommen O und f' eine Zahl ist, die von der Wertigkeit und Anzahl der Atome der anderen Elemente bestimmt wird, wobei d' + e' eine Zahl von 0,01 bis 5 ist.

8. Verfahren nach Anspruch 7, dadurch gekennzeichnet, daß der Katalysator Heteropolysäurestruktur hat.

9. Verfahren nach Anspruch 7, dadurch gekennzeichnet, daß a' = 10, b' eine Zahl von 0,5 bis 3, c' eine Zahl von 0,5 bis 3, d' eine Zahl von 0,01 bis 1,0 und e' eine Zahl von 0,01 bis 1,0 ist.

10. Verfahren nach Anspruch 9, dadurch gekennzeichnet, daß der Katalysator Heteropolysäurestruktur hat.